(19) Europäisches Patentamt
European Patent Office
Office européen des brevets

(11) **EP 4 451 180 A1**

(12) **EUROPEAN PATENT APPLICATION**

(43) Date of publication:
**23.10.2024 Bulletin 2024/43**

(21) Application number: **23305614.2**

(22) Date of filing: **20.04.2023**

(51) International Patent Classification (IPC):
***G06N 10/40*** *(2022.01)* ***G06N 10/60*** *(2022.01)*
***G16B 15/00*** *(2019.01)* *G06N 5/01 (2023.01)*
*G06N 7/01 (2023.01)*

(52) Cooperative Patent Classification (CPC):
**G06N 10/60; G06N 10/40; G16B 15/20; G16B 15/30;** G06N 5/01; G06N 7/01

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Pasqal S.A.S.**
  **91300 Massy (FR)**
- **Qubit Pharmaceuticals S.A.S.**
  **75014 Paris (FR)**
- **SORBONNE UNIVERSITE**
  **75006 Paris (FR)**
- **Centre National de la Recherche Scientifique**
  **75016 Paris (FR)**

(72) Inventors:
- **PIQUEMAL, Jean-Philip**
  **75052 Paris (FR)**
- **LOCO, Daniele**
  **75014 Paris (FR)**
- **GOURAUD, Nicolaï**
  **75014 Paris (FR)**
- **d'ARCANGELO, Mauro**
  **91300 MASSY (FR)**
- **HENRY, Louis-Paul**
  **91300 MASSY (FR)**

(74) Representative: **De Vries & Metman**
**Overschiestraat 180**
**1062 XK Amsterdam (NL)**

(54) **DETERMINING A LOCATION OF A MOLECULAR ENTITY AT AN INTERACTION SITE OF A MACROMOLECULE USING A QUANTUM COMPUTER**

(57) Methods for determining a location of a molecular entity at an interaction site of a protein using a quantum computer and hybrid computing systems for executing such methods are described wherein the method includes encoding a problem of determining a location of a molecular entity at an interaction site of a macromolecule, such as a protein, into a problem Hamiltonian, the encoding including mapping a density distribution of molecular entities at an interaction site of a protein onto a plurality of qubits of the quantum computer, each qubit being associated with a location in the density distribution and defining a possible location of a molecular entity at the interaction site; computing pulse parameters for generating one or more control signals for configuring the quantum computer in a state of the problem Hamiltonian; evolving the qubits of the quantum computer based on the control signals and measuring the state of the quantum computer; and, determining one or more candidate locations for a molecular entity at the interaction site based on the measured state.

encoding a protein hydration problem into a problem Hamiltonian by mapping the density function of a hydrated protein onto a spatial arrangement of neutral atoms forming a quantum register, each neutral atom defining a possible position of a water molecule in a cavity of the protein
302
computing pulse parameters for generating laser pulses for configuring the quantum register in the ground state of an initial Hamiltonian and for evolving the ground state of the initial Hamiltonian to a ground state of the problem Hamiltonian
304
preparing the quantum register in a ground state of an initial Hamiltonian and evolving the ground state of the initial Hamiltonian to the ground state of the problem Hamiltonian based on the laser pulses
306
measuring Rydberg excitations in the quantum processor and storing the measurements
308
determining the ground state of the problem Hamiltonian based on the measurements and determining the position of the water molecules in the hydrated protein based on the ground state
310

Fig. 3

EP 4 451 180 A1

## Description

### Technical field

The disclosure relates to determining a location of a molecular entity at an interaction site, in particular molecular interaction sites, of a macromolecule, such as a protein, using a quantum computer, and in particular, though not exclusively, to methods and systems for determining a location of a molecular entity at an interaction site of a macromolecule using a quantum computer and quantum computer program products for determining such interaction sites.

### Background

The problem of finding locations of molecular entities at interaction sites of a macromolecule, for example locations of water molecules forming the most stable water network configuration inside occluded cavities of a protein, is an important problem in drug discovery. Various numerical methods have been developed to address this problem, and among these the 3D reference interaction site model (3D-RISM) presents a good compromise between execution time and accuracy. The shortcoming of 3D-RISM is that it outputs a continuous density distribution function rather than exact discrete locations at which molecules, such as water molecules, are positioned within the cavities of a protein. The exact location of the molecules within a cavity of a macromolecule is hereafter referred to as an interaction site.

Software packages for translating the 3D-RISM density into explicit positions are known. For example, GAsol, as described in the article by Fusani et al, Optimal water networks in protein cavities with GAsol and 3D-RISM, Bioinformatics, 34(11), 2018, 1947-1948, uses a genetic algorithm where the cavity is discretized on a lattice and different configurations (i.e. different placements of the water molecules) compete, mate, reproduce and mix with each other. GAsol imposes a penalty to configurations that are too dissimilar from the 3D-RISM density, or where the water molecules are too close to each other (because of some physical constraint). The process of competition and reproduction is iterated many times, until in the end the best configuration is selected.

The location of the interaction sites in a protein cavity is governed by physical phenomena such as the Van der Waals interaction between water molecules, which forbids two molecules to be too close to each other. Although GAsol uses a heuristic to deal with configurations in which water molecules are close to each other, it does not provide an accurate model representing the physical processes at molecular scale. Hence, there is a need in the art for improved methods and system for determining a location of a molecular entity at an interaction site of a macromolecule.

### Summary

As will be appreciated by one skilled in the art, aspects of the present invention may be embodied as a system, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Functions described in this disclosure may be implemented as an algorithm executed by a microprocessor of a computer. Furthermore, aspects of the present invention may take the form of a computer program product embodied in one or more computer readable medium(s) having computer readable program code embodied, e.g., stored, thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber, cable, RF, etc., or any suitable combination of the foregoing.

Computer program code for carrying out operations for aspects of the present invention may be written in any combination of one or more programming languages, including an object-oriented programming language such as Java(TM), Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described below with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer program instructions. These computer program instructions may be provided to a processor, in particular a microprocessor or central processing unit (CPU), of a general purpose computer, special purpose computer, or other programmable data processing apparatus to produce a machine, such that the instructions, which execute via the processor of the computer, other programmable data processing apparatus, or other devices create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks. Additionally, the Instructions may be executed by any type of processors, including but not limited to one or more digital signal processors (DSPs), general purpose microprocessors, application specific integrated circuits (ASICs), field programmable logic arrays (FP- GAs), or other equivalent integrated or discrete logic circuitry.

The flowchart and block diagrams in the figures illustrate the architecture, functionality, and operation of possible implementations of systems, methods and computer program products according to various embodiments of the present invention. In this regard, each block in the flowchart or block diagrams may represent a module, segment, or portion of code, which comprises one or more executable instructions for implementing the specified logical function(s). It should also be noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustrations, and combinations of blocks in the block diagrams and/or flowchart illustrations, can be implemented by special purpose hardware-based systems that perform the specified functions or acts, or combinations of special purpose hardware and computer instructions.

The embodiments in this disclosure aim to determine interaction sites in a macromolecule, such as a protein, using a quantum computer. In particular, the method and systems described in this disclosure are configured to determine potential molecule positions using a qubit register, such as an array of neutral atoms, from which the best placement of molecules in a macromolecule cavity may be determined through analog quantum computing using the information given by a distribution function as input.

In an aspect, the embodiments may relate to a method for determining a location of a molecular entity at an interaction site of a protein using a quantum computer. The method may include encoding a problem of determining a location of a molecular entity at an interaction site of a macromolecule, such as a protein, into a problem Hamiltonian. The encoding may include mapping a density distribution of molecular entities at an interaction site of a protein onto a plurality of qubits of the quantum computer. Each qubit may be associated with a location in the density distribution and define a possible location of a molecular entity at the interaction site.

The method may further include computing parameters for generating one or more control signals for configuring the quantum computer in a state of the problem Hamiltonian; evolving the qubits of the quantum computer based on the control signals and measuring the state of the quantum computer; and, determining one or more candidate locations for a molecular entity at the interaction site based on the measured state.

Hence, the inventors surprisingly found that the general problem of finding locations of an molecular entity at an site interaction site in a cavity of a macromolecule, such as a protein, based on a continuous density function may be encoded into an Ising model, in particular an anti-ferromagnetic model (AFIM), whose solution is an NP-hard problem in classical computing. This way, the problem can be addressed by quantum computing schemes.

In particular, the embodiments in this application provide methods and systems which are configured to efficiently compute locations of a molecular entity at an interaction site in a cavity of a macromolecule, by encoding the problem as an Ising model and computing a solution to the problem in the quantum domain using a quantum computer. One particular relevant use case relates to finding locations of water molecules at a site interaction site in a cavity of a protein. The algorithms at least substantially reduce the use of expensive classical long Molecular Dynamics or Monte Carlo simulations.

In an embodiment, the plurality of qubits may include a spatial arrangement of qubits. Such arrangement may include 1D, 2D or 3D spatial arrangement of qubits.

In an embodiment, the molecular entity may be a water molecule.

In an embodiment, the one or more control signals may be configured to set the quantum computer in an initial state and to evolve the initial state towards a final state, preferably a ground state or a low-energy state of the problem Hamiltonian.

In an embodiment, the problem Hamiltonian may be an Ising-type Hamiltonian. In another embodiment, the Hamiltonian may approximate an Ising-type Hamiltonian.

In an embodiment, the method may further include a quantum optimization scheme.

In an embodiment, the quantum optimization may include: constructing a distribution model, the construction including assigning a model distribution function to each of the one or more candidate positions; determining a similarity measure based on the density distribution and the distribution model; optimize the one or more candidate locations based on the similarity measure by updating the pulse parameters, evolve the qubits based on the updated parameters and measuring one or more updated candidate locations.

The 3D Reference Interaction Site Model (3D-RISM) or a related model, which may be used to predict continuous distributions, may be used by a variational quantum algorithm (VQA) as described with reference to the embodiments in this application to learn the most favorable, in a thermodynamics sense, discrete configuration of solvent molecules.

Here, a VQA is an algorithm, which uses a classical optimizer to find the parameters of a control field, e.g. a laser pulse, that provides an approximate ground state which is a approximate solution of the problem Hamiltonian

Different algorithms may be used to perform the optimization procedure required to find the optimal parameters to physically encode the problem's solution into the qubits' quantum state. The quantum annealing and VQA schemes exhibit great potential to predict the solvation structure in molecular systems of great interest for applications in drug discovery. The embodiments provide a new domain of applications for Rydberg atom quantum machines.

The VQA process may be fully performed on a classical computer and the obtained optimized parameters can be directly fed to a quantum processor to generate the target distributions.

In an embodiment, the model distribution function may be one of: a Gaussian distribution, Lorentzian distribution, a radial basis function, a isotropic model distribution function or anisotropic model distribution function.

In an embodiment, a qubit may be associated with a first state indictive of no presence of a molecular entity and a second state indicative of the presence of a molecular entity.

In an embodiment, the qubits may be atomic qubits.

In an embodiment, an atomic qubit includes a Rydberg state indicating a position of a molecular entity at the interaction site.

Hence, the Hamiltonian of a system of Rydberg atoms may be leveraged to map the information of a density function of molecules in a protein, such as a 3D-RISM density or an experimentally determined density function, into the best placement of molecules inside a cavity of a macromolecule. In an embodiment, the molecules may be water molecules. An accurate prediction of the location of molecules can facilitate drug discovery processes.

In an embodiment, the problem Hamiltonian may be a Rydberg Hamiltonian.

In an embodiment, the control signals may be one or more laser pulses.

In an embodiment, the one or more control signals may be one or more local laser pulses for individually addressing an atomic qubit.

In an embodiment, the one or more control signals may be one or more global pulses for collectively addressing a plurality of atomic qubits of the quantum computer.

In an embodiment, the method may comprise: measuring the state of the atomic qubits in the computational basis using an imaging system.

In an embodiment, the measured state of the atomic qubits may form a bitstring for encoding the one or more candidate locations.

In an embodiment, the evolution of the qubits of the quantum computer may be based on a quantum annealing scheme or based on a variational quantum algorithm.

In a further aspect, the embodiments may relate to a hybrid computing system for determining a location of a molecular entity at an interaction site of a protein comprising a quantum computer comprising a plurality of qubits, preferably a spatial arrangement of atomic qubits, forming a quantum register and a classical computer.

In an embodiment, the system may be configured to perform one or more of the following steps: encoding a problem of determining a location of a molecular entity at an interaction site of a macromolecule, such as a protein, into a problem Hamiltonian, the encoding including mapping a density distribution of molecular entities at an interaction site of a protein onto a plurality of qubits of the quantum computer, each qubit being associated with a location in the density distribution and defining a possible location of a molecular entity at the interaction site; computing pulse parameters for generating one or more control signals for configuring the quantum computer in a state of the problem Hamiltonian; evolving the qubits of the quantum computer based on the control signals and measuring the state of the quantum computer; and, determining one or more candidate locations for a molecular entity at the interaction site based on the measured state.

In an embodiment, the hybrid computing system may be further configured to perform any of the method steps described above.

In yet a further aspect, the invention may relate to a computer program or suite of computer programs comprising at least one software code portion the software code portion, when run on a hybrid computing system comprising a plurality of qubits, preferably a spatial arrangement of atomic qubits, forming a quantum register and a classical computer, being configured for executing the method steps according to any of the embodiments described

## Brief Description of the drawings

**Fig. 1** depicts an example of a 3D-RISM density map;
**Fig. 2A** and **2B** depict an example of a density function of cavities of a protein in which water molecules are located;
**Fig. 3** depicts a method for determining hydration sites of a protein according to an embodiment;
**Fig. 4** depicts a method for determining hydration sites of a protein according to another embodiment;
**Fig. 5** schematically depicts mapping regions of a 2D density function onto a 2D lattice of neutral atoms of a quantum register;
**Fig. 6** schematically depicts construction of a density function based on Gaussians placed around excited atoms of a trial state of the quantum processor.
**Fig. 7** depicts a density function and the location of water molecules which were obtained using a variational algorithm according to an embodiment;
**Fig. 8** depicts a histogram of states that were sampled during the execution of the variational algorithm;
**Fig. 9** depicts a hybrid computer system comprising a classical computer and a quantum processor;
**Fig. 10** schematically describes an example of a neutral atom quantum processor;
**Fig. 11A-11D** depict implementations of quantum spin models using neutral atoms.

## Description of the embodiments

Macromolecules, such as proteins, have a complicated structure with crevices and cavities forming potential binding sites for external ligands. For example, solvent molecules (water) play an important role in the binding process. Understanding the hydration of protein cavities has applications in drug design and discovery. In particular, knowledge about the amount of water molecules and the location of water molecules in protein cavities is important information. Numerically, molecular dynamics (MD) or Monte Carlo (MC) computations may provide accurate predictions, but suffer from extremely long simulation time and/or computational expensive computations, if a cavity is occluded enough.

Another solution is to use 3D-RISM (Reference Interaction Site Model), which is related to continuum models that view the solvent as a continuous dielectric, but it maintains to some degree a microscopic description of the solvent molecules. 3D-RISM is considerably faster than brute-force schemes based on MD and MC and works well with occluded cavities. The downside is that it outputs a continuous density, for example in case of water molecules oxygen density, without explicit information about the number of water molecules as well as the location of the water molecules in the cavity.

The 3D-RISM model is based on an integral-equation formalism, which allows computing the solvent density around a given solute (e.g., a protein) based on a number of model parameters. The output of a 3D-RISM calculation is a function $g(\vec{r})$ that to each point $\vec{r}$ assigns a non-negative quantity, representing the water density in that position.

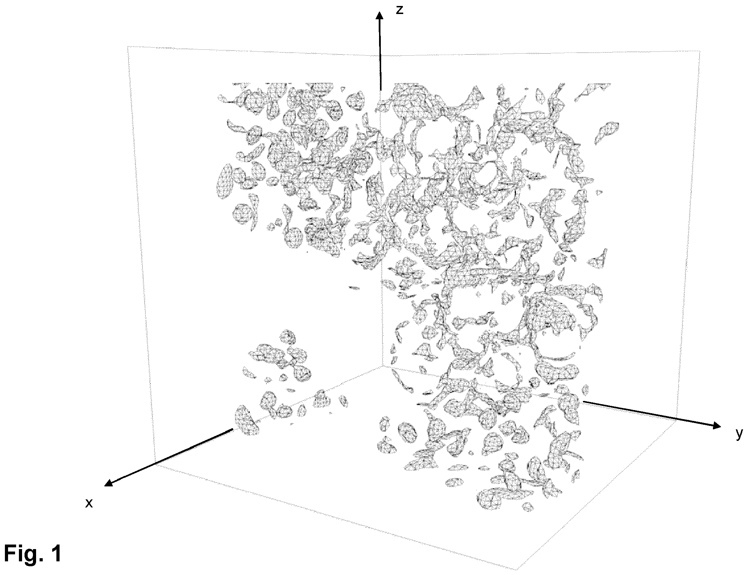

An example of 3D-RISM density is shown in **Fig. 1** which shows iso-surfaces of value 4 in a 3D space. For many purposes, it is desired to know the explicit discrete position of each molecule in the pocket instead of their joint density distribution.

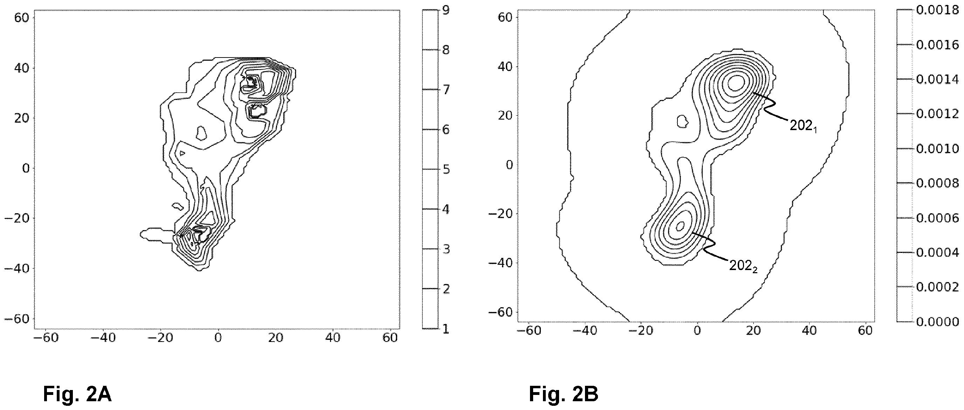

**Fig. 2A** depicts an example of part of a density function, in this case a 2D slice of a 3D density function of a cavity of the protein MUP1. This protein is known to have just two water molecules inside the cavity. A density function may also be determined experimentally. For example, a density function may be determined based on crystallographic measurements, for example X-Ray crystallography of electron densities, of a hydrated protein. It is customary to apply a Laplacian of Gaussian (LoG) filter to the densities to smooth them out and emphasize the maximum density values

**$202_{1,2}$** in a filtered density as shown in **Fig. 2B.**

The exact locations of the water molecules however typically do not coincide with the maximum density values. Moreover, without prior knowledge about the exact number of water molecules, it is not possible to determine whether the density is the result of two molecules or maybe three or even four molecules. Additionally, it is not possible to determine whether an individual density peak is the result of one or more water molecules. Hence, explicit positions of the molecules need to be determined based on a computed or measured density of one or more protein cavities without prior knowledge about number of molecules that interact with the protein. The general form of the problem of finding positions of $N$ molecules, e.g. water molecules, in a protein based on a density function $g(\vec{r})$ may be defined as a minimization problem wherein a distance $D$ is minimized under the constraint that the distance between two molecules is larger than some critical distance R, which defined a sphere in which two molecules at positions $\vec{w}_t$ and $\vec{w}_j$ cannot simultaneously be present: $|\vec{w}_i - \vec{w}_j| > R \ \forall \ i,j$ (equation 1):

$$\begin{cases} \{\vec{w}_i^{best}\} = \arg\min_{\{\vec{w}_i\}} \ D(g(\vec{r}), \{\vec{w}_i\}) \\ \text{subject to: } \ |\vec{w}_i - \vec{w}_j| > R \quad \forall \ i,j \end{cases}$$

It is noted that this scheme is not only suitable for determining the locations of water molecules at interaction sites in a cavity, but can be generally used to determine locations of molecular entities, e.g. molecules, atoms, ions, ligands at interaction sites of a macromolecule. Hence, although the embodiments in this application are described with reference to water molecules at interaction sites of a protein cavities, they are not limited thereto. For example, 3D-RISM also allows determination of ion-distributions around nucleic acids.

A quantum computer comprising a qubit register is particularly suitable for finding an optimal global solution for the problem defined above with reference to equation (1). In particular, an array of atomic qubits is particularly suitable for finding an optimal global solution to the problem by using the Rydberg blockade mechanism. Hence, the embodiments in this application aim to provide efficient and accurate quantum algorithms which provide an accurate estimate for positions of molecules in cavities of macromolecules, such as proteins. In particular, the embodiments aim to provide methods and systems for determining interaction sites in a protein using a quantum processor, which outputs for a given density $g(\vec{r})$ a set of $M$ positions $\{\vec{w}_i\}$, $i$ = 1,..., $M$, corresponding to the positions of $M$ molecules in the protein cavity.

Before translating the classical problem description into a quantum problem description, the distance function $D(g(\vec{r}), \{\vec{w}_i\})$ may be defined which can be used to compare a classically computed or measured density with a density model that is based on positions of interaction sites of a protein cavity. Different distances may be used for example, the $L^2$ norm, the total variation distance, the Kullback-Leibler divergence or the Jensen-Shannon distance.

The problem of determining interaction sites may be approached by interpreting the density as the result of a certain number of molecules oscillating around a stable position within a protein cavity. In that case, the joint density may be decomposed into a plurality of single-particle densities expressing the uncertainty in the position of each water molecule. In an embodiment, the single-particle densities may have a certain distribution, for example a Gaussian distribution, a Lorentzian distribution or a radial basis function. Such distribution may be isotropic distribution or non-isotropic distribution.

A protein cavity may be modeled as a connected subset

$$\mathcal{C} \subset \mathbb{R}^3 ;$$

and a density, such as a computed 3D-RISM, may be regarded as a positive scalar function

$$g: \mathcal{C} \to \mathbb{R}^+.$$

Further, in case an isotropic Gaussian $\mathcal{G}(\vec{\mu}, \sigma^2)$ is used to describe the individual molecules, such distribution may be characterized by a mean $\vec{\mu}$ and variance $\sigma^2$. The possible values of the mean positions of the Gaussians may be discretized according to a discrete grid of points in space $Q = \{\vec{q}_1, \ldots, \vec{q}_N\} \subset \mathcal{G}$. Further, each point in the space may be associated with a binary occupation variable $n_i \in \{0,1\}$ so that an arbitrary sum of Gaussians can be written as follows (equation 2):

$$\sum_{i=1}^{N} \mathcal{G}(\vec{q}_i, \sigma^2) n_i.$$

The sum of Gaussians may also be referred to as a Gaussian mixture and a model based on a Gaussian mixture may be referred to as a Gaussian mixture model (GMM). The value of each occupation variable $n_i$ will act as a "switch" that indicates whether or not a Gaussian is present at position $\vec{q}_i$. Then, the problem can be formulated as finding the optimal set of variables of $\{n_i\}_{i=1,...,N}$ and $\sigma^2$ such that a distance function $D(g, GMM)$ which defines a "distance" between $g$ and GMM is minimized.

For example, in an embodiment, the $L^2$ norm may be used as a distance. In that case, the expression to be minimized is defined by the following integral over the cavity volume C (equation 3):

$$I^2 := \int_C \left( g(\vec{r}) - \sum_{i=1}^{N} \mathcal{G}(\vec{q}_i, \sigma^2)(\vec{r})\ n_i \right)^2 d\vec{r}$$

Hence, in this embodiment, a computed or measured target density $g$ may be compared with a mixture of Gaussians which are arranged on a discrete grid of points. The comparison may be based on a similarity measure such as the $L^2$ distance between distribution $g$ and a distribution based on a Gaussian mixture as defined in equation 2. Expanding the square in equation 3 one can derive that $I^2$ defines the energy of a classical Ising model (equation 4):

$$I^2 = K - \sum_{i=1}^{N} \Gamma_i n_i + \sum_{i \neq j = 1}^{N} V_{ij} n_i n_j$$

where $K$ is an unimportant constant, and the coefficients of the linear and quadratic terms are given by the coefficients $\Gamma_i$ and $V_{ij}$ which are defined as integrals over the cavity volume $\mathcal{C}$ (equation 5):

$$\Gamma_i := 2 \int_C g(\vec{r})\ \mathcal{G}(\vec{q}_i, \sigma^2)(\vec{r})\ d\vec{r} - \int_C \left( \mathcal{G}(\vec{q}_i, \sigma^2)(\vec{r}) \right)^2 d\vec{r}$$

and (equation 6):

$$V_{ij} := \int_C \mathcal{G}(\vec{q}_i, \sigma^2)(\vec{r})\ \mathcal{G}(\vec{q}_j, \sigma^2)(\vec{r})\ d\vec{r}$$

The coefficients may be computed numerically. It is however insightful to further examine the interaction term $V_{ij}$. When the Gaussians are concentrated far enough from the boundary of the cavity $\mathcal{C}$, it is possible to extend the integration to the whole of the $\mathbb{R}^3$ space. By performing the change of variables $\vec{r} \rightarrow \vec{r} + \vec{q}_i$, the term becomes the convolution of two Gaussians centered in zero, which is itself a Gaussian. In that case, the interaction term $V_{ij}$ can be approximated by an exponential term (equation 7):

$$V_{ij} \sim \exp\left(-\alpha |\vec{r}_{ij}|^2\right).$$

wherein $\vec{r}_{ij} = \vec{q}_j - \vec{q}_i$. Equation (3) thus represents a classical Ising model with exponentially decaying interactions. So if $\{n_1^*, \dots, n_N^*\}$ is the ground state of the Ising model, the solution to the water placement problem is then given by a number of water molecules $M := \sum_{i=1}^{N} n_i^*$ having positions corresponding to those $\vec{q}_i$ for which $n_i^* = 1$ (equation 8):

$$W = \{ \vec{q}_i \in Q | \; n_i^* = 1\}$$

The quantum version of the Ising model may be obtained by replacing the binary variable $n_i$ with the number operator $\hat{n}_i$, whose spectrum is {0,1}. In terms of Pauli matrices, the number operator may be defined as follows $\hat{n}_i = (\hat{\sigma}_i^z + 1)/2$ . Taking the classical Ising model of equation (3) and performing the substitution $n_i \rightarrow \hat{n}_i$, the Hamiltonian of a system of interacting spins located in $\vec{q}_1, \dots, \vec{q}_N$ is obtained (equation 9):

$$I^2 \rightarrow \hat{I}^2 := -\sum_{i=1}^{N} \Gamma_i \hat{n}_i + \sum_{i \neq j=1}^{N} V_{ij} \hat{n}_i \hat{n}_j$$

This Hamiltonian may be referred to as the problem Hamiltonian. The ground state of the problem Hamiltonian corresponds to the ground state of the classical Ising model of equation (3), but it is now seen as the computational basis vector $|e^*\rangle$ in the Hilbert space of the spin system that minimizes the expectation value of the problem Hamiltonian (equation 10):

$$|e^*\rangle = \min_{|\psi\rangle \in \mathcal{H}} \langle \psi | \hat{I}^2 | \psi \rangle .$$

The number $M$ and the set of positions $W$ of the molecules may then be given by the following expressions (equations 11 and 12):

$$M := \sum_{i=1}^{N} \langle e^* | \hat{n}_i | e^* \rangle$$

$$W = \{\vec{w}_1, \dots, \vec{w}_M\} := \{\vec{q}_i \mid \; \langle e^* | \hat{n}_i | e^* \rangle = 1\}.$$

It is noted however that minimization of the Ising energy (as defined by equation 10) alone is not enough, as the problem statement according to equation (1) is constrained by a minimum distance between the placed water molecules $|\vec{w}_i - \vec{w}_j| > R \; \forall \; i,j$.

Hence, the problem of finding molecular interaction sites in a protein cavity may be solved by determining the ground state of the problem Hamiltonian. A quantum computer comprising a qubit register is particularly suited for determining the ground state of a problem Hamiltonian. Although the embodiments hereunder are described based on a neutral atom quantum processor, other quantum processors based on other hardware platforms such as superconducting qubits or trapped ion qubits may also be used.

In an embodiment, a quantum computer comprising a quantum register implemented based on an array of atomic qubits may be used to determine the ground state of the problem Hamiltonian.

An advantage of solving the protein hydration problem using such neutral atom quantum processor is that the neutral atom system evolves according to a Hamiltonian similar to the problem Hamiltonian, and the minimum distance constraint is automatically satisfied. The neutral atoms of the quantum computer may be addressed by optical signals of an optical laser system such that the state of the quantum processor evolves according to the so-called Rydberg Hamiltonian (equation 13):

$$\hat{H}(t) = \sum_{i=1}^{M} \Omega_i(t)\hat{\sigma}_i^x - \sum_{i=1}^{M} \Delta_i(t)\hat{n}_i + \sum_{i<j=1}^{M} U_{ij}\hat{n}_i\hat{n}_j$$

where $U_{ij}$ is the interaction coefficient between Rydberg excitations (equation 14):

$$U_{ij} = \frac{C_6}{r_{ij}^6}$$

and $C_6$ is a physical constant, and $r_{ij}$ is the distance between two atoms $r_{ij} = |\vec{q}_i - \vec{q}_j|$ wherein $\vec{q}_i$ is the position of atom i. Comparison of both Hamiltonians (9) and (13) shows that the Rydberg Hamiltonian is similar to the problem Hamiltonian except for the interaction term. The difference between the problem Hamiltonian and the Rydberg Hamiltonian resides in the coefficient of the interaction term, which in case of the Rydberg Hamiltonian $U_{ij}$ decays as a power-law, while in the problem Hamiltonian $V_{ij}$ decays exponentially.

The problem Hamiltonian of equation (9) does not directly encode the proximity constraint between neighboring excitations. In contrast the Van der Waals term, i.e. the $r^6$ term, of the Rydberg Hamiltonian intrinsically provides such proximity constraint. In particular, the Van der Waals interaction term of the Rydberg Hamiltonian forbids two neighboring atoms in the ground state $|00\rangle$ to be excited simultaneously, thus producing the superposition state $|01\rangle + |10\rangle$ instead. This is known as the Rydberg blockade mechanism, which is the main source of entanglement in neutral atom-based quantum computers. Hence, the solution provided by the neutral atom quantum processor will never provide solutions of water molecules positions that are located very close to each other. Such constraint is imposed by hand in classical approaches such as GAsol.

In an embodiment, a neutral atom quantum processor, may be configured as a Rydberg quantum processor. In that case, each atom may represent a physical qubit associated with a number or occupation operator $\hat{n}_i$ having eigenstates 10) and $|1\rangle$ wherein 10) is the ground state of the atoms, while 11) is a Rydberg state of an atom. By optically addressing an atom with a predetermined laser beam, a transition from the ground state 10) to the Rydberg state $|1\rangle$ may be initiated. This way, each atom effectively represents a qubit associated with a two-dimensional Hilbert space. The atomic system that is configured based on the Rydberg Hamiltonian, evolves in time according to the Schrödinger equation. The time-dependent coefficients $\Omega_i(t)$ and $\Delta_i(t)$ of the Rydberg Hamiltonian may be controlled using programmable control parameters of the quantum processor. Here, $\Omega_i(t)$ corresponds to Rabi frequency and $\Delta_i(t)$ corresponds to the detuning of the driving laser respectively that is used for addressing the atoms. After evolving the atomic qubits for a period of time $T$, the system can be measured in the computational basis using a known fluorescence imaging process for distinguishing atoms in the $|0\rangle$ state and in the excited Rydberg $|1\rangle$ state. The positions of the atoms in the Rydberg state $|1\rangle$ may be interpreted as the location of water molecules in the cavity.

Thus, the Rydberg Hamiltonian of the neutral atom quantum processor may be controlled based on parameters that can be externally controlled, using e.g. lasers and/or microwave control signals. In particular, the coefficients $\Omega_i(t)$ and $\Delta_i(t)$ of the Rydberg Hamiltonian may be controlled using the Rabi frequency and detuning of the driving laser respectively.

In an embodiment, the neutral atom quantum computer may be used as an analog quantum computer wherein the solution to a problem, such as the problem of determining interaction sites, may be determined using a quantum annealing scheme. The ground state of a Hamiltonian, such as the problem Hamiltonian, may be found by slowly evolving the ground state of a time-dependent Hamiltonian over a period of time $T$ from an initial Hamiltonian $H(0)$ to a final Hamiltonian $H(T)$, wherein $H(T)$ corresponds to the problem Hamiltonian or approximates the problem Hamiltonian.

In an embodiment, the initial Hamiltonian $\hat{H}(0)$ may be based on the Rydberg Hamiltonian. For example, for the initial Hamiltonian the Rydberg Hamiltonian may be used wherein the coefficients are selected to be zero for all atoms (i.e. $\Omega_i(0) = \Delta_i(0) = 0$ for all $i$). This ground state $|0...0\rangle$ can be easily prepared experimentally. In another embodiment, the so-called equal superposition state may be used as the initial sate. The final Hamiltonian $H(T)$ may be based on the Rydberg Hamiltonian, wherein $\Omega_i(t') = 0$, $\Delta_i(t') = \Gamma_i$ approximates the problem Hamiltonian. In that case, each node of the problem Hamiltonian may have different weights determined by coefficients $\Gamma_i$, which can be computed classically.

The physical scale of the problem Hamiltonian (e.g. molecules in a protein cavity) and the Rydberg Hamiltonian (e.g. array of atomic qubits) may differ substantially. In that case, the original one-body coefficients $\Gamma_i$ may be related to the detunings $\Delta_i$ by a transformation of the type $\Delta_i = \alpha\Gamma_i + \beta$. The purpose of $\alpha$ is to rescale the units of the original problem to match those of the Rydberg Hamiltonian, while $\beta$ is a constant shift that could help in aligning the low-energy spectrum of the two Hamiltonians. The procedure presented below is not exact, but rather aimed at providing an efficient heuristic to find the best $\alpha$ and $\beta$ for a particular application.

The complexity in finding the ground state of the problem Hamiltonian (equation 9) lies in the competition between the one-body and two-body terms, where spins in 11) are energetically favoured by the one-body terms, but energetically disfavoured by the two-body terms. It is then reasonable to expect that the Rydberg Hamiltonian (equation 13) better approximates the low-energy spectrum of the problem Hamiltonian if the relationship between $\Gamma_i$ and $V_{ij}$ is well reproduced in the relationship between $\Delta_i$ and $U_{ij}$. This condition may be expressed in different ways (equation 15-17):

$$\sum_{i,j=1}^{m} \frac{\Gamma_i \Gamma_j}{V_{ij}} \approx \sum_{i,j=1}^{m} \frac{\Delta_i \Delta_j}{U_{ij}}$$

$$\sum_{i,j=1}^{m} \frac{V_{ij}}{\Gamma_i \Gamma_j} \approx \sum_{i,j=1}^{m} \frac{U_{ij}}{\Delta_i \Delta_j}$$

$$\sum_{i,j=1}^{m} \Delta_i \Delta_j V_{ij} \approx \sum_{i,j=1}^{m} \Gamma_i \Gamma_j U_{ij}$$

The quantity to be computed is the square of the logarithmic difference. Taking the second relation (equation 16) as an example, the logarithmic difference $T$ may be computed as (equation 18):

$$T(\alpha, \beta)^2 := \left[ \log \left| \sum_{i,j=1}^{m} \frac{V_{ij}}{\Gamma_i \Gamma_j} \right| - \log \left| \sum_{i,j=1}^{m} \frac{U_{ij}}{\Delta_i \Delta_j} \right| \right]^2$$

Minimizing $T$ will give an optimal solution for $\alpha$ and $\beta$. Based on these parameters the one-body coefficients $\Gamma_i$ may be transformed into one or more control signals (for example one or more optical detuning signals $\Delta_i$) for locally controlling qubits of a quantum register.

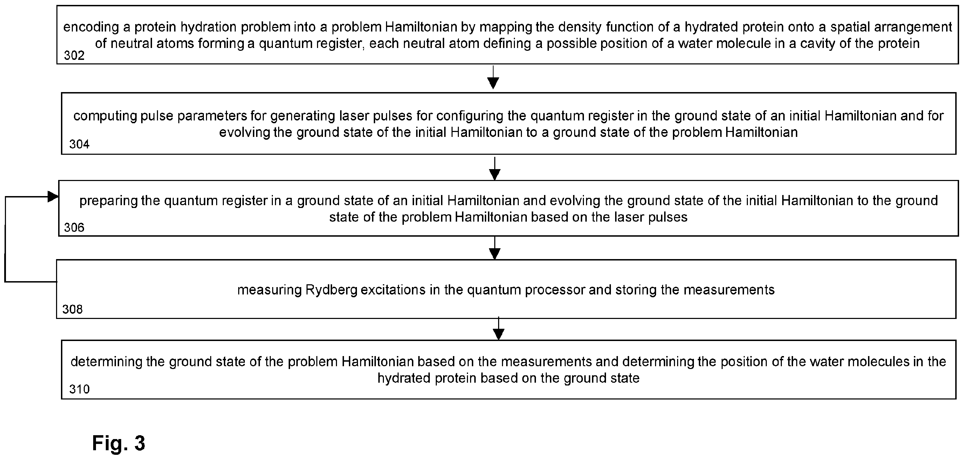

**Fig. 3** schematically depicts method for determining interaction sites in a protein cavity according to an embodiment. In particular, the figure depicts a flow chart of a method for determining interaction sites based on a quantum annealing scheme. In that case, the neutral atom quantum register may be configured as a quantum annealer. The quantum annealer may be controlled by a classical computer which is configured to encode the problem into a problem Hamiltonian and to compute pulse parameters for generating laser pulses for executing a quantum annealing scheme.

Hence, the method may include encoding the problem of determining the interaction sites into a problem Hamiltonian (step **302**). The encoding may include mapping a density function associated with the protein onto a spatial arrangement of neutral atoms forming the neutral atom quantum register (in shorth the quantum register) wherein each neutral atom may define a possible position of a water molecule. The method may also comprise computing pulse parameters for a laser system for generating laser pulses for configuring the quantum register in the ground state of an initial Hamiltonian and for evolving the ground state of the initial Hamiltonian to a ground state or low-energy state of the problem Hamiltonian (step **304**).

The laser pulses may be used to prepare the quantum register into a ground state of an initial Hamiltonian and to evolve the ground state of the initial Hamiltonian to the ground state or low-energy state of the problem Hamiltonian (step **306**).

Thereafter, Rydberg excitations in the quantum register may be measured (step **308**). The measurement may include determining a bitstring wherein each bit in the bitstring represents an atom of the neutral atom register. Here, an excited atom may be represented a first binary value (e.g. "1") and an atom that is not in the Rydberg state may be represented by a second binary value (e.g. "0"). The measured bitstring may be stored to repeat the process. The initialization and evolution of the quantum register may be repeated N times to determine a set of measurements that allows to determine the ground state of the problem Hamiltonian (step **310**).

Hence, the quantum register may be initialized into an initial Hamiltonian and slowly controlled to evolve the initial Hamiltonian into the problem Hamiltonian. The annealing process may be controlled based on tunable parameters of the problem Hamiltonian, in particular the coefficients $\Gamma_i$ of the problem Hamiltonian. These tunable parameters may be computed and includes the computation of detuning parameters $\Delta_i(t)$ which may be used to generate optical pulse signals for optically addressing the qubits during annealing. A data acquisition system is used to measure the states of atoms of the quantum processor. In particular, the classical computer may determine a trial solution to the computational problem based on the measured information and verify the quality of the trial solution. For *NP* problems, the verification is a computation which can be carried out in polynomial time, and can typically be easily computed. This process may be repeated until a satisfactory solution to the computational problem is found.

The above-described quantum annealing approach requires locally (individually) addressing the atoms of the neutral atom processor, which may be problematic for quantum registers comprising a very large number of atoms.

In a further embodiment, instead of the Rydberg Hamiltonian as defined by equation 13, a global Rydberg Hamiltonian may be used, wherein the coefficients $\Omega$ and $\Delta$ are no longer local parameters, but global parameters (equation 19):

$$\hat{H}(t) = \frac{\hbar\Omega(t)}{2}\sum_{i=1}^{M}\hat{\sigma}_i^x - \hbar\Delta(t)\sum_{i=1}^{M}\hat{n}_i + \sum_{i<j=1}^{M}\frac{C_6}{r_{ij}^6}\hat{n}_i\hat{n}_j.$$

Thus, when using the global Rydberg Hamiltonian, one or more global laser pulses acting in the same way on every individual atom (i.e. global addressing) may be used to evolve the atomic quantum processor to a ground state. In general, there is insufficient overlap between the low-energy spectrum of the problem Hamiltonian and the low-energy spectrum of the global Rydberg Hamiltonian. Hence, when only global addressing is available, an annealing scheme as described with reference to **Fig. 3** cannot be used.

To overcome this problem, a hybrid quantum-classical variational algorithm may be used which is configured to finding the global parameters $\Omega$(t) and $\Delta$(t) that maximize the overlap between the quantum state obtained at the end of time evolution and the ground state of the problem Hamiltonian. Hence, in this embodiment, a hybrid quantum classical formulation of the problem can be used to define the problem as an optimization problem, for example an argmin problem.

For example, in an embodiment, a Bayesian search routine may be used wherein the quantum evolution to reach $|\psi_T\rangle_k$, $k$ = 1, ..., $n_c$ is repeated $n_c$ times to explore paths in the $\Omega$-$\Delta$ plane. At the end of the $k$-th repetition, the resulting state $|\psi_T\rangle_k$ may be measured several times and the bitstring $|b_i\rangle_k$ may be recorded that best minimize a scoring function score ($\langle b_i|I^2|b_i\rangle_k$). At the end of the $n_c$ cycles, a histogram of the bitstrings $|b_i\rangle$ may be generated to finally extract the expected one with highest probability of occurrence.

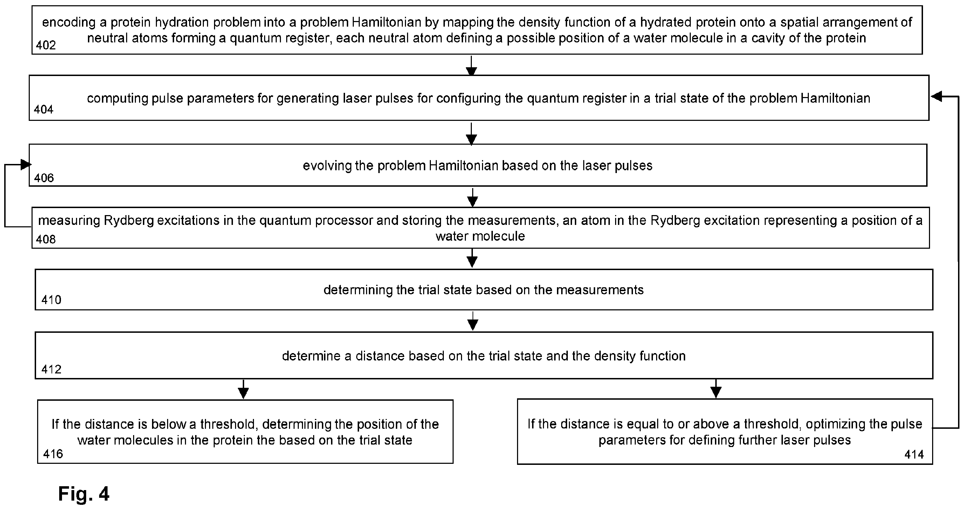

**Fig. 4** schematically depicts a variational quantum algorithm for determining interaction sites in a protein cavity according to an embodiment. In particular, the figure depicts a flow diagram of a method for determining interaction sites in a protein cavity using a variational algorithm and global optical addressing of the neutral atoms. The method may include encoding the problem of determining the interaction sites into a problem Hamiltonian (step **402**). In an embodiment, the problem Hamiltonian may be based on the Rydberg Hamiltonian, for example a global Rydberg Hamiltonian, wherein the coefficients $\Omega$ and $\Delta$ are no longer local parameters, but global parameters.

The encoding may include mapping a density function of a hydrated protein cavity onto a spatial arrangement of neutral atoms forming a quantum register, wherein each neutral atom may define a possible position of a water molecule in a cavity of the protein. Further, pulse parameters may be computed for an optical system to generate laser pulses, preferably laser pulses for globally addressing the atoms of the quantum processor. The pulses may be determined to configure the neutral atom quantum register in a trial state of the problem Hamiltonian. In an embodiment, the pulse parameters may be based on the detuning parameter of the problem Hamiltonian. Further, the laser pulses may be used to evolve the problem Hamiltonian into a trial state of the quantum register, wherein atoms in the Rydberg state may represent locations of water molecules.

Rydberg excitations in the quantum register may be measured (step **408**) using a suitable imaging scheme. Similar to the method described with reference to **Fig. 3,** the measurement may generate a bitstring, wherein each bit in the bitstring represents an atom in of the quantum register and wherein an excited atom may be represented a first binary value (e.g. "1") and an atom that is not in the Rydberg state may be represented by a second binary value (e.g. "0"). The measured bitstring may be stored and the formation and measurement of trial states of the problem Hamiltonian (produced by the laser pulses) may be repeated N times to determine a set of measurements that can be used to determine a final trial state (step **410**).

A distance representing a similarity metric may be determined based on the final trial state and the density function (step **412**). The final trial state may include a set of excited atoms at certain positions in the neutral atom quantum register. Each excited atom may be associated with a Gaussian distribution so that the sum of the Gaussians may form a density function that can be compared with the target density function of the hydrated protein. The distance may define a metric for determining the similarity (or overlap) between the two functions. A threshold value may be determined if the two functions are sufficiently similar. Hence, if the distance is equal to or above this threshold, then the pulse parameters may be optimized to define new laser pulses for configuring the quantum register in a new trial state (step **414**). This new trial state can be measured and evaluated based on the distance in a similar way as described above. This process may be repeated until the distance is below the threshold. In that case, the optimized trial state may be used to determine the position of the water molecules (step **416**).

In an embodiment, the distance may be based on a Gaussian mixture model. For example, the Rabi frequency $\Omega$ and detuning of the driving laser $\Delta$ for addressing the atoms may be used to control the Rydberg Hamiltonian of the quantum processor. The Rabi frequency $\Omega$ and detuning of the driving laser $\Delta$ for addressing the atoms may be used to control the Rydberg Hamiltonian of the quantum processor.

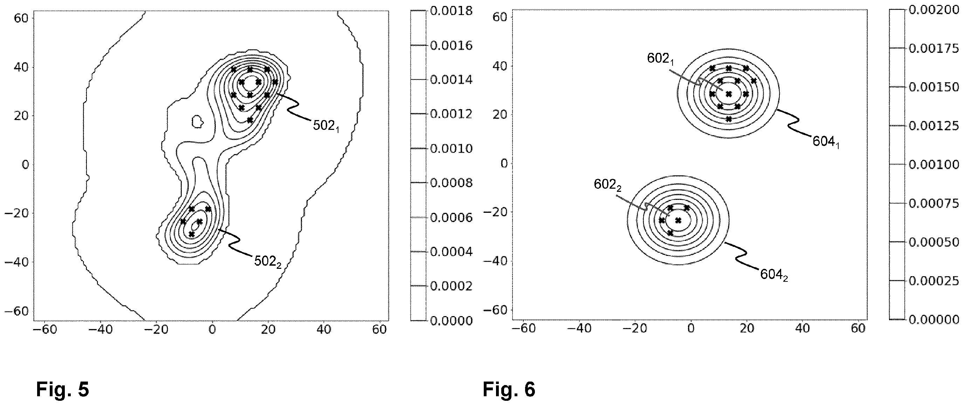

**Fig. 5** schematically depicts mapping regions **$502_{1,2}$** of a 2D density function onto a 2D lattice of qubits, e.g. atomic qubits, of a quantum register illustrated by the crosses in the density. For clarity reasons only a limited number of qubits are illustrated. The illustrated qubits may be selected to represent potential candidates for the position of interaction sites, e.g. locations of water molecules, in a protein cavity. If the control parameters that control the time evolution of the qubits are set correctly, there is a high probability that the system evolves to a state wherein qubits that are close to the most probable position of an interaction site will be in state 11), while the other qubits remain in the ground state 10). Thus, when measuring for example a final state $|00101\rangle$ a bitstring 00101 may be produced representing a configuration of interaction sites wherein an interaction site may be positioned - in this example - at the position of the third and fifth neutral atom.

As explained above, atomic qubits, i.e. qubits based on neutral atoms, are particularly suited for determining interaction sites inside a protein cavity on the basis of a density function because there is a minimal physical distance between molecules. For water molecules this distance is given approximately by the average length of a hydrogen bond. Rydberg excitations in a atomic qubit quantum register follow a similar constraint, wherein two atoms too close to each other cannot be excited simultaneously because of the Rydberg blockade.

The optimal control parameters $\Omega$ and $\Delta$ for the evolution of the quantum system can be found in different ways. Typically, the Hamiltonian of the quantum system may be associated to a cost function representing the problem, with the optimal parameters of the Hamiltonian being the ones that minimize the cost function. In an embodiment, such a cost function may be based on comparison of the original distribution, e.g. a 3D-RISM, with configurations of water molecules returned by the quantum evolution. The different nature of the two objects (the original distribution function is a continuous map while the interaction sites are represented by a discrete list of positions) makes the comparison non-trivial. To make this comparison possible, in an embodiment, a single particle distribution function, e.g. a Gaussian may be placed around each excited atom (representing an interaction site) to form a mixture of Gaussians. This way, the distance metric is based on a comparison between the density function and a mixture of Gaussians.

For example, an evolution of the qubits based on a density function of **Fig. 5** may yield a bitstring: 0100000010000000, indicating an interaction site close to qubit 2 and an interaction site close to qubit 9. The Gaussian mixture related to that bitstring is depicted in **Fig. 6,** which is constructed by placing a Gaussian **$604_{1,2}$** around the second and ninth qubit **$602_{1,2}$**. For a certain value of the control parameters $\Omega$ and $\Delta$, the result of measuring ten times the system in **Fig. 5** may yield the following samples:

| measurement | state | frequency |
|---|---|---|
| 1 | 0100000100010000 | 3 |
| 2 | 0100000010000000 | 3 |
| 3 | 0100010000100000 | 2 |
| 4 | 0100000000100000 | 1 |
| 5 | 0100010000010000 | 1 |

A certain score associated with parameters $\Omega$ and $\Delta$ may be used to define a distance between the original 3D-RISM density and the distribution of Gaussians, for example a weighted average of the Gaussian mixture, associated to the sampled bitstrings. The weight of each Gaussian mixture may be given by the frequency with which that bitstring was sampled. This score may be used as an objective function that can be minimized using a minimization scheme, e.g. a Bayesian minimization scheme, to select good parameters $\Omega$ and $\Delta$.

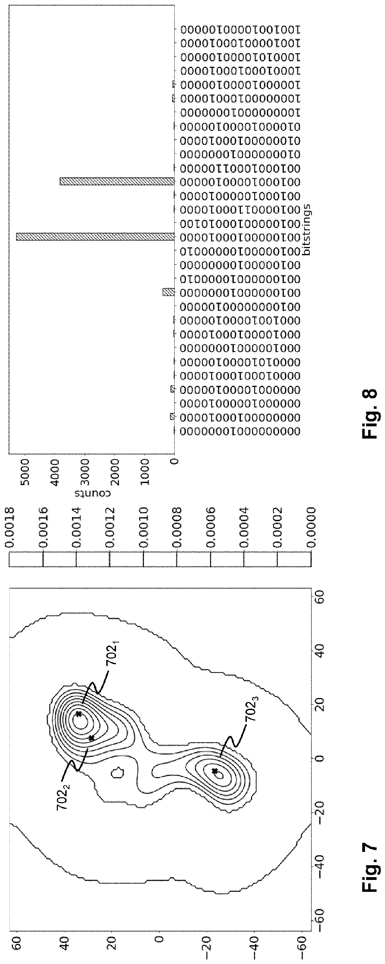

Applying this scheme to the density of **Fig. 5** using a limited number of qubits (N = 17) already provides good results. **Fig. 7** depicts a density function and locations of three interaction sites **$702_{1-3}$** which were obtained based on a Bayesian search of the optimal Hamiltonian parameters as explained above. The resulting Gaussian mixture nicely approximates the original density of **Fig. 5. Fig. 8** depicts a histogram of states that were sampled during the process. The most sampled state 0100000100010000 corresponds to the best solution to the problem. This result also clearly illustrates that it is not possible to derive the location of the interaction sites solely based on the density of **Fig. 5.**

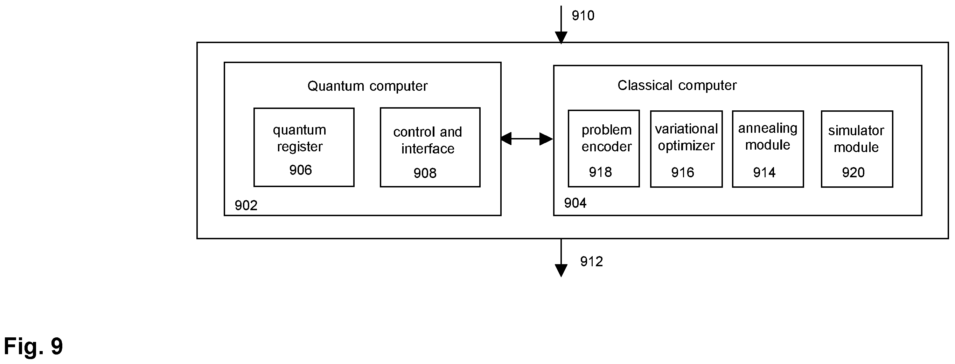

Generally, the schemes described in this application may be executed by a hybrid computer system as depicted **in Fig. 9.** Such hybrid computer system may comprise a classical computer **904** comprising one or more classical processors (CPUs and/or GPUs) and a quantum processor **902** comprising a quantum register **906** comprising a plurality of interacting quantum elements, e.g. qubits or qudits, that can be controlled by the classical computer. Here, the qubits may be implemented based on different types of technologies, including but not limited to superconducting qubits, quantum dot qubits, neutral atom type qubits, optical qubits, etc.

The quantum processor may be controlled via a controller system **908** comprising input output (I/O) devices which form an interface between the quantum processor and classical computer. The controller system may include a system for generating control signals for controlling the quantum processing elements. The control signals may include for example pulses, e.g. microwave pulses, voltage pulses and/or optical pulses, for manipulating the qubits, e.g. bringing them in an initial state and manipulating the state of the qubit and/or the interaction (coupling) between two or more qubits. Further, the controller may include a data acquisition system for readout of state of the quantum processing elements. Hence, the data acquisition system may receive an output signal originating from the quantum processor in response to a readout pulse for reading the state of a quantum element. In some embodiments, at least a part of the data acquisition system may be located or integrated with the chip that includes the qubits.

The classical computing system may include different modules for enabling execution of programs, in particular quantum annealing algorithms and variational quantum algorithms, to be executed on the quantum processor. To that end, the classical computer may include a problem encoder **918** configured to receive a computational problem such as the protein hydration problem and to encode the problem into a problem Hamiltonian of the quantum processor. Additionally, the classical computer may include a variational optimizer module **916** for executing variational quantum algorithms and/or an annealing module **914** for executing quantum annealing schemes.

The classical computer may include an emulator module **920** to emulate classically the quantum dynamics. The emulator module may include exact solvers like state vector, Schrodinger equation and master equation solvers, for exact emulation of a limited number of qubits. The classical emulator may also include approximate solvers like tensor network algorithms and/or deep neural networks to approximately emulate the dynamics of a limited number of qubits.

The problem Hamiltonian may be an Ising-type Hamiltonian, such as a Rydberg type Hamiltonian, based on Pauli operators acting on a k-th qubit of the qubits of the quantum processor. The problem Hamiltonian may also include one or more adjustable parameters which can be controlled by external electromagnetic control signals, e.g. electromagnetic, optical and/or magnetic fields and/or pulses, which may be used to locally and/or globally adjust the electromagnetic or magnetic environment at one or more qubits. A plurality of such adjustable external control signals may be used to control a single qubit or a part of the plurality of qubits.

By adjusting the external control signals, these parameters may be adjusted depending on the computational problem. Encoding the computational problem into the problem Hamiltonian, as performed by the classical computing system, includes determining, from the computational problem, a configuration for the plurality of adjustable parameters. For each of the adjustable parameters may be determined depending on the computational problem.

The controller system may include a data acquisition system configured to measure the states of at least part of the qubits of the quantum processor, wherein the measured information may include at least part of the solution to the problem that is encoded in the problem Hamiltonian. In particular, the classical computer may determine a trial solution to the computational problem based on the measured information, and verify if the trial solution actually is a solution to the computational problem. For NP problems, the verification is a computation which can be carried out in polynomial time, and can typically be easily computed. This process may be repeated until a solution to the computational problem is found.

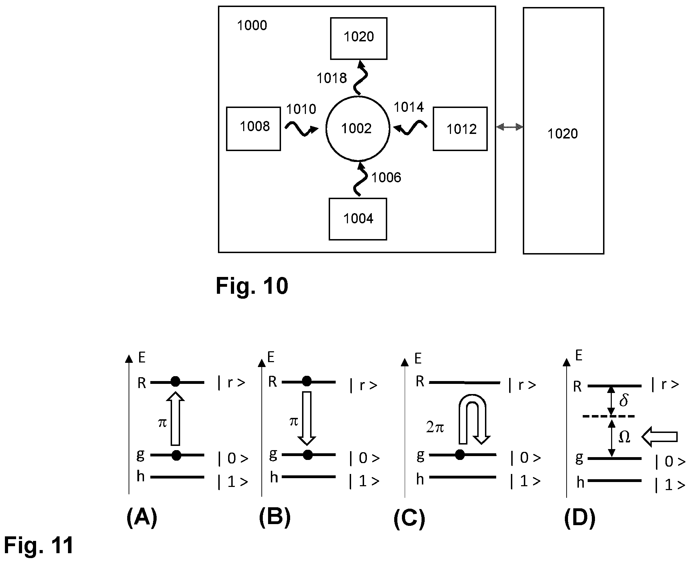

One particular advantageous hybrid computer system for implementing and executing schemes described in this application, is a neutral atom quantum processor based on configurable arrays of single neutral atoms (also referred to as a neutral atom register). **Fig. 10** schematically describes an example of such neutral atom quantum processor. In particular, the figure depicts a high-level schematic of a neutral atom quantum processor **1000** (quantum processor) which is controlled by a classical computer **1020** and which is configured to execute the quantum circuits as described with reference to the embodiments in this application.

The quantum computer may include a chamber **1002** that accommodates a plurality of neutral atoms. The atoms may be of the same element, and thus are, from a chemical standpoint, identical when no external interactions are imposed upon them. The atoms may be unbound to other atoms in the group, for example, by being in a gaseous matter state. Particular suitable atoms that can be used as qubits and which are suitable for trapping, positioning and atomic-state-manipulating may include (but not limited to) Rubidium or Cesium or Strontium (Alkali, Alkaline Earth, ...).

To control the quantum processor to execute operations, it may include different control and readout modules as shown in the figure. In particular, the quantum processor may include amongst others a trapping system **1004** configured to trap atoms in a particular spatial arrangement within the chamber, an atom positioner **1008** configured to controllably move one or more trapped atoms from one spatial position to another spatial position, an atomic state actuator **1012** or in short an actuator configured to generated optical control pulses to control and manipulate the atomic states of atoms in the chamber, and a detector **1020** configured to detect and capture optical signals **1018** transmitted by the atoms in the chamber. The detector may comprise a camera to image the fluorescence output by the atoms held by the holding system.

The trapping system **1004** may be configured to trap atoms in a particular spatial arrangement. In particular, the trapping system may be configured to position (trap) each atom of the group of atoms at a particular position in the chamber such that they form a predetermined spatial arrangement in which atoms are isolated from each other if they are in a non-excited state, while atoms within a certain region may interact if they are in an excited state. Hence, the term 'isolate' in this context means that an atom in a non-excited atomic state does not interact with a neighbouring atom of the same group. However, if the atoms are stimulated using, for example, an electromagnetic signal such as a laser pulse, they may be brought into an excited state, in which the atoms may interact with each other based on quantum mechanical effects such as (but not limited to) the Rydberg blockade.

The trapping system may be configured to trap one atom at each site so that a spatial arrangement of single atoms is formed wherein each atom forms a multi-level quantum system that can be used as a qubit. In another embodiment, the trapping system may be configured to configured hold multiple atoms, e.g. two or three, at each site. Each set of multiple atoms may form a multi-level quantum system that can be configured as a qubit (or a qudit).

The trapping system may be configured to maintain the atoms in their stationary positions using different mechanisms including, but not limited to, magnetically traps and optical traps. The trapping system may be configured to generate a pattern of spatially separated traps so that a particular spatial arrangement of atoms in the chamber can be realized. The trapping pattern may be an array of regular or irregular spaced traps. The trapping patterns may include 1D, 2D (insofar that the traps in the pattern all align along one plane) or 3D patterns of traps. For example, the trapping system may generate a 3D array of traps spaced periodically in the X, Y and Z dimensions to form a 3D grid. Other patterns are also possible. The spacing in one spatial dimension may be the same or different to the other spatial dimensions.

The trapping system may provide a plurality of trapping sites wherein, when the trapping system is first activated some trapping sites may be filled by one or more atoms whilst other trap sites are vacant. Preferably the trapping system may be configured to generate trapping sites that hold a single atom. The trapping system may use electromagnetic signals **1006,** such as optical trapping signals to generate the optical traps in the chamber.

The atom positioner **1008** may be configured to controllably move one or more held atoms from one spatial position to another spatial position. For example, in an embodiment, the atom positioner may include one or more optical tweezers configured to use optical signals **1010** to move one or more trapped atoms in one of the trapping sites to another trapping site. Different technologies may be used to manipulate the position of the atoms including, but not limited to, moving the atoms using magnetic signals or electromagnetic signals such as optical signals.

The atomic state actuator **1012** may be configured to generate optical pulse signals to control and manipulate the atomic states of atoms in the chamber (in other words it "actuates" the transition between atomic states). The optical pulse signal **1014** may include single or multiple photon signals. Different optical pulse signals may be output by the atomic state actuator including optical pulse signals at different wavelengths. Each wavelength may correspond to (i.e., be resonant with) a different atomic transition. Typically, the quantum processor may comprise multiple atomic state actuators. For example, a first atomic state actuator may output a first wavelength or first set of wavelengths which are different from the wavelength or set of wavelengths outputted by a second atomic state actuator. The atomic state actuator may facilitate the transition between atomic states of a single trapped atom or a plurality of trapped atoms. The wavelengths may be selected based on the atoms in the chamber. The excitation from the ground state to the Rydberg state may be facilitated by two-photon absorption. This may be accomplished using two different EM sources such as lasers or other EM sources. These two EM sources may have different wavelengths. For example, optical control pulses of 495 nm may be used for exciting a Rubidium atom to the Rydberg state and optical control pulses of 795 nm to induce transitions between the hyperfine states.

Suitable signals for trapping and moving the atoms are preferably different, at least in wavelength, to the signals used to manipulate the quantum states of the atoms. In particular, signals for trapping and moving the atoms may be off-resonance, i.e., the wavelength of the optical signals for trapping and positioning an atom cannot excite the atom between its different atomic states.

An example of the general operation of the quantum processor may include one or more of the following steps.

1) Using the trapping system to generated optical trapping signals to create a plurality of traps in the chamber so that atoms in the chamber are trapped.
2) Optionally using the atom positioner to manipulate, e.g. move, trapped atoms so that each trap of at least a predetermined set of traps can be filled with a single atom. Such set of single atom filled traps may be referred to as a 'register'. The detector may be used in this process to help identify which traps are occupied or vacant.
3) Using the atomic state actuator to generate predetermined optical control pulses **1014,** e.g. laser pulses of a predetermined shape, amplitude and duration to control the atomic states of atoms in the register. This step may be performed multiple times to implement processing operations of the quantum processor, for example, time-sequentially inputting a plurality of optical pulses that represent quantum logic gate operations.
4) Using the detector to detect and image florescent signals emitted by the atoms and using the imaged signals to determine the atomic states of the atoms.

These steps may represent a quantum computation by the quantum processor. The quantum processor may be reset by removing the traps and re-initialised for a further quantum computation by repeating steps 1-4 above.

Fig. 11A-11D depict implementations of quantum spin models using neutral atoms. In particular, Fig. 11A-11C schematically show three levels of the atomic system of the rubidium atoms used herein wherein: the energy levels '*g*' and '*h*' denote the hyperfine states that represent the $|0\rangle$ and $|1\rangle$ qubit states respectively, whilst '*R*' represents the Rydberg state of the atomic system and is associated with quantum state $|r\rangle$. The label of '$\pi$' in **Fig. 11A** and **11B** is shown when a single transition is made between the *g* and *R* state indicating that a $\pi$ phase change has been imparted into the atomic system because of the overall transition.

When a laser field of sufficient duration and amplitude has been imparted onto the atom to resonantly transition it from the *g* energy level up to the *R* energy level and then back to the *g* energy level (within the same control field input), this is labelled as '$2\pi$' indicating that a $2\pi$ phase change has been imparted into the atomic system because of the overall transition. The laser fields that give rise to these $\pi$ and $2\pi$ transitions may be respectively referred to as a $\pi$-pulse and a $2\pi$-pulse. It is understood that the input control fields causing these transitions have a wavelength resonant with the $|0\rangle$ to $|r\rangle$ transition and not resonant between the $|0\rangle$ and $|1\rangle$ transition. **Fig. 11A** shows the atomic transition from the *g* level to the *R* level with a $\pi$-pulse. **Fig. 11B** shows the atomic transition from the *R* level to the *g* level with a $\pi$-pulse. **Fig. 11C** shows the atomic transition from the *g* level to the *R* level and back to the *g* level again with a $2\pi$-pulse.

**Fig. 11D** depicts a near-resonant laser pulse of amplitude $\Omega$ exposing an atom. The frequency of the laser pulse is detuned from the transition frequency between the ground state $|g\rangle$ and a Rydberg state $|r\rangle$ by a small (with respect to the transition frequency) detuning $\delta$. The amplitude $\Omega$ of the pumping laser determines the transverse-field term in the Ising model, and the detuning to resonance $\delta$ induces a longitudinal-field term. Additionally, the global phase of the laser can be tuned in order to control the axis of rotation on the Bloch sphere induced by the transverse-field term. The pulse depicted in **Fig. 11D** represents a single gate operation defining a predetermined rotation over the Block sphere.

Various components, modules, or units are described in this disclosure to emphasize functional aspects of devices configured to perform the disclosed techniques, but do not necessarily require realization by different hardware units. Rather, as described above, various units may be combined in a hardware unit or provided by a collection of interoperative hardware units, including one or more processors as described above, in conjunction with suitable software and/or firmware.

The terminology used herein is for the purpose of describing particular embodiments only and is not intended to be limiting of the invention. As used herein, the singular forms "a," "an," and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description, but is not intended to be exhaustive or limited to the invention in the form disclosed. Many modifications and variations will be apparent to those of ordinary skill in the art without departing from the scope and spirit of the invention. The embodiment was chosen and described in order to best explain the principles of the invention and the practical application, and to enable others of ordinary skill in the art to understand the invention for various embodiments with various modifications as are suited to the particular use contemplated.

## Claims

**1.** Method for determining a location of a molecular entity at an interaction site of a macromolecule, such as protein, using a quantum computer:

encoding a problem of determining a location of a molecular entity at an interaction site of a macromolecule, such as a protein, into a problem Hamiltonian, the encoding including mapping a density distribution of molecular entities at an interaction site of a protein onto a plurality of qubits of the quantum computer, each qubit being associated with a location in the density distribution and defining a possible location of a molecular entity at the interaction site;

computing parameters for generating one or more control signals for configuring the quantum computer in a state of the problem Hamiltonian;

evolving the qubits of the quantum computer based on the control signals and measuring the state of the quantum computer; and,
determining one or more candidate locations for a molecular entity at the interaction site based on the measured state.

**2.** Method according to claim 1 wherein the plurality of qubits include a spatial arrangement of qubits, preferably a 1D, 2D or 3D spatial arrangement of qubits.

**3.** Method according to claims 1-2 wherein the molecular entities are water molecules.

**4.** Method according to any of claims 1-3 wherein the one or more control signals are configured to set the quantum computer in an initial state and to evolve the initial state towards a final state, preferably a ground state or a low-energy state of the problem Hamiltonian.

**5.** Method according to any of claims 1-4 wherein the problem Hamiltonian is an Ising-type Hamiltonian or a Hamiltonian that approximates an Ising-type Hamiltonian.

**6.** Method according to any of claims 1-5 wherein the method further includes:

constructing a distribution model, the construction including assigning a model distribution function to each of the one or more candidate positions;
determining a similarity measure between the density distribution and the distribution model;
optimize the one or more candidate locations based on the similarity measure by updating the pulse parameters, evolve the qubits based on the updated parameters and measuring one or more updated candidate locations.

**7.** Method according to claim 6 wherein the model distribution function is one of a Gaussian distribution, Lorentzian distribution, a radial basis function, a isotropic model distribution function or anisotropic model distribution function.

**8.** Method according to any of claims 1-7 wherein a qubit is associated with a first state indictive of no presence of a molecular entity and a second state indicative of the presence of a molecular entity.

**9.** Method according to claim 8 wherein the qubits are atomic qubits, preferably an atomic qubit in a Rydberg state indicating a position of a molecular entity at the interaction site; and/or, wherein the problem Hamiltonian is a Rydberg Hamiltonian.

**10.** Method according to any of claims 1-9 wherein the control signals are one or more laser pulses, preferably one or more local laser pulses for individually addressing an atomic qubit and/or one or more global pulses for collectively addressing a plurality of atomic qubits of the quantum computer.

**11.** Method according to any of claims 1-10 wherein the method comprises: measuring the state of the atomic qubits in the computational basis using an imaging system, preferably the measured state of the atomic qubits forming a bitstring for encoding the one or more candidate locations.

**12.** Method according to any of claims 1-11 wherein the evolution of the qubits of the quantum computer are based on a quantum annealing scheme or based on a variational quantum algorithm.

**13.** A hybrid computing system for determining a location of a molecular entity at an interaction site of a protein comprising a quantum computer comprising a plurality of qubits, preferably a spatial arrangement of atomic qubits, forming a quantum register and a classical computer, wherein the system is configured to perform the steps of:

encoding a problem of determining a location of a molecular entity at an interaction site of a macromolecule, such as a protein, into a problem Hamiltonian, the encoding including mapping a density distribution of molecular entities at an interaction site of a protein onto a plurality of qubits of the quantum computer, each qubit being associated with a location in the density distribution and defining a possible location of a molecular entity at the interaction site;
computing parameters for generating one or more control signals for configuring the quantum computer in a state of the problem Hamiltonian;
evolving the qubits of the quantum computer based on the control signals and measuring the state of the quantum computer; and,

determining one or more candidate locations for a molecular entity at the interaction site based on the measured state.

**14.** A hybrid computing system according to claim 13, wherein the hybrid computing system is further configured to perform any of the steps according to claims 2-12.

**15.** A computer program or suite of computer programs comprising at least one software code portion the software code portion, when run on a hybrid computing system comprising a plurality of qubits, preferably a spatial arrangement of atomic qubits, forming a quantum register and a classical computer, being configured for executing the method steps according any of claims 1-12.

y
z
x

Fig. 1

$202_1$
$202_2$
0.0018
0.0016
0.0014
0.0012
0.0010
0.0008
0.0006
0.0004
0.0002
0.0000
9
8
7
6
5
4
3
2
1

Fig. 2A

Fig. 2B

encoding a protein hydration problem into a problem Hamiltonian by mapping the density function of a hydrated protein onto a spatial arrangement of neutral atoms forming a quantum register, each neutral atom defining a possible position of a water molecule in a cavity of the protein
302
computing pulse parameters for generating laser pulses for configuring the quantum register in the ground state of an initial Hamiltonian and for evolving the ground state of the initial Hamiltonian to a ground state of the problem Hamiltonian
304
preparing the quantum register in a ground state of an initial Hamiltonian and evolving the ground state of the initial Hamiltonian to the ground state of the problem Hamiltonian based on the laser pulses
306
measuring Rydberg excitations in the quantum processor and storing the measurements
308
determining the ground state of the problem Hamiltonian based on the measurements and determining the position of the water molecules in the hydrated protein based on the ground state
310

Fig. 3

encoding a protein hydration problem into a problem Hamiltonian by mapping the density function of a hydrated protein onto a spatial arrangement of neutral atoms forming a quantum register, each neutral atom defining a possible position of a water molecule in a cavity of the protein
402
computing pulse parameters for generating laser pulses for configuring the quantum register in a trial state of the problem Hamiltonian
404
evolving the problem Hamiltonian based on the laser pulses
406
measuring Rydberg excitations in the quantum processor and storing the measurements, an atom in the Rydberg excitation representing a position of a water molecule
408
determining the trial state based on the measurements
410
determine a distance based on the trial state and the density function
412
If the distance is equal to or above a threshold, optimizing the pulse parameters for defining further laser pulses
414
If the distance is below a threshold, determining the position of the water molecules in the protein the based on the trial state
416

Fig. 4

$502_1$
$502_2$
$602_1$
$602_2$
$604_1$
$604_2$
0.0018
0.0016
0.0014
0.0012
0.0010
0.0008
0.0006
0.0004
0.0002
0.0000
0.00200
0.00175
0.00150
0.00125
0.00100
0.00075
0.00050
0.00025
0.00000
60
40
20
0
−20
−40
−60


Fig. 5

Fig. 6

702₁
702₂
702₃
counts
bitstrings


Fig. 7

Fig. 8

910
912
Classical computer
904
problem encoder
918
variational optimizer
916
annealing module
914
simulator module
920
Quantum computer
902
quantum register
906
control and interface
908

Fig. 9

1000
1002
1004
1006
1008
1010
1012
1014
1018
1020
1020
E
R
g
h
| r >
| 0 >
| 1 >
π
2π
δ
Ω
(A)
(B)
(C)
(D)

Fig. 10

Fig. 11

Europäisches Patentamt
European Patent Office
Office européen des brevets

# EUROPEAN SEARCH REPORT

Application Number
EP 23 30 5614

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MOHIT PANDEY ET AL: "Multibody molecular docking on a quantum annealer", ARXIV.ORG, CORNELL UNIVERSITY LIBRARY, 201 OLIN LIBRARY CORNELL UNIVERSITY ITHACA, NY 14853, 20 October 2022 (2022-10-20), XP091349261, * abstract; figures 1-2 * * Sections I-II, III.A, and IV * ----- | 1-15 | INV. G06N10/40 G06N10/60 G16B15/00 ADD. G06N5/01 G06N7/01 |
| A | MORGADO M ET AL: "Quantum simulation and computing with Rydberg-interacting qubits", ARXIV.ORG, CORNELL UNIVERSITY LIBRARY, 201 OLIN LIBRARY CORNELL UNIVERSITY ITHACA, NY 14853, 30 December 2020 (2020-12-30), XP081848327, * the whole document * ----- | 1-15 | |
| A | MICHEL ANTOINE ET AL: "Blueprint for a digital-analog variational quantum eigensolver using Rydberg atom arrays", PHYSICAL REVIEW A, [Online] vol. 107, no. 4, 6 April 2023 (2023-04-06), XP093084210, ISSN: 2469-9926, DOI: 10.1103/PhysRevA.107.042602 Retrieved from the Internet: URL:https://journals.aps.org/pra/pdf/10.1103/PhysRevA.107.042602> [retrieved on 2023-09-21] * the whole document * ----- -/-- | 1-15 | TECHNICAL FIELDS SEARCHED (IPC) G06N G06F G16C G16B |

The present search report has been drawn up for all claims

1

| Place of search | Date of completion of the search | Examiner |
|---|---|---|
| The Hague | 22 September 2023 | Cilia, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt
European
Patent Office
Office européen
des brevets

# EUROPEAN SEARCH REPORT

Application Number
EP 23 30 5614

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YUICHIRO YOSHIDA ET AL: "Solvent distribution effects on quantum chemical calculations with quantum computers", ARXIV.ORG, CORNELL UNIVERSITY LIBRARY, 201 OLIN LIBRARY CORNELL UNIVERSITY ITHACA, NY 14853, 26 August 2022 (2022-08-26), XP091303498, * the whole document * ----- | 1-15 | |
| A | LOIC HENRIET ET AL: "Quantum computing with neutral atoms", ARXIV.ORG, CORNELL UNIVERSITY LIBRARY, 201 OLIN LIBRARY CORNELL UNIVERSITY ITHACA, NY 14853, 22 June 2020 (2020-06-22), XP081700350, * the whole document * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 22 September 2023 | Cilia, Elisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04C01)

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **FUSANI et al.** Optimal water networks in protein cavities with GAsol and 3D-RISM. *Bioinformatics,* 2018, vol. 34 (11), 1947-1948